# EUROPEAN PATENT APPLICATION

(11) **EP 4 282 955 A1**
(43) Date of publication of application: **29.11.2023**
(21) Application number: 21921200.8
(22) Date of filing: 16.11.2021
(51) Int. Cl.: C12N 7/00, C12N 7/01

(54) **VIRUS STABILIZER, GELATIN HYDROLYSATE FOR VIRUS STABILIZER, AND VIRUS-CONTAINING COMPOSITION**

(30) Priority: 19.01.2021 JP 2021006669
(71) Applicant: Nitta Gelatin Inc., Osaka 556-0022 (JP); Incorporated Educational Institution Rakuno Gakuen, Ebetsu-shi, Hokkaido, 069-8501 (JP)
(72) Inventor: HAGIWARA, Katsuro, Ebetsu-shi, Hokkaido 069-8501 (JP); IDA, Masataka, Yao-shi, Osaka 581-0024 (JP); HIRAOKA, Yosuke, Yao-shi, Osaka 581-0024 (JP); NGAKO KADJI, Francois Marie, Yao-shi, Osaka 581-0024 (JP); KOTANI, Kazuki, Yao-shi, Osaka 581-0024 (JP); TSUKAMOTO, Hiroshi, Yao-shi, Osaka 581-0024 (JP)
(74) Representative: Vossius & Partner Patentanwälte Rechtsanwälte mbB
(86) International application number: PCT/JP2021/042018
(87) International publication number: WO 2022/158097

(57) **Abstract**

The virus stabilizer is a virus stabilizer comprising a gelatin hydrolysate and an aqueous medium, wherein the virus stabilizer contains the gelatin hydrolysate at not less than 1 mass% and not more than 20 mass%, the gelatin hydrolysate has a weight average molecular weight of not more than 10000, and the gelatin hydrolysate has an isoelectric point pH of not less than 4.0 and not more than 7.0.

## Description

### TECHNICAL FIELD

The present invention relates to a virus stabilizer, a gelatin hydrolysate for a virus stabilizer, and a virus-containing composition.

### BACKGROUND ART

For storage and transportation of viruses, an environment of -80 to -60°C is generally required for maintaining viral capabilities such as infectivity or preventing a decrease in the viral capabilities. When a virus is stored and transported at room temperature, it is necessary that a preparation containing the virus be a freeze-dried product. Thus, storage and transportation of viruses have been heretofore known to be troublesome. Meanwhile, Japanese Patent Laying-Open No. 2013-035861 (Patent Literature 1) discloses a refrigerator-stable influenza virus composition that is stable at a refrigerated temperature (e.g. 2 to 8°C).

### CITATION LIST

### PATENT LITERATURE

PTL 1: Japanese Patent Laying-Open No. 2013-035861

### SUMMARY OF INVENTION

### TECHNICAL PROBLEM

Patent Literature 1 discloses that with a composition containing a 0.5 to 2% gelatin hydrolytic product and the like, stable storage and transportation of an influenza virus strain at a refrigerated temperature were achieved. However, Patent Literature 1 does not disclose whether or not it is possible to achieve stable storage and transportation of the virus strain at a room temperature of about 25°C. According to Patent Literature 1, it is considered that since the gelatin hydrolytic product is obtained from porcine gelatin type A having an isoelectric point of about 9, the gelatin hydrolytic product strongly interacts with a specific protein on the viral surface, so that it is difficult to stably store and transport the virus at room temperature. Further, it is known that when a preparation or the like containing a virus which is stored in an environment at -80 to -60°C is heated for solation and is returned to a refrigerated temperature (2 to 8°C) or room temperature for use, a decrease in viral capabilities such as infectivity cannot be avoided. Materials have not been provided yet which does not require a complicated operation and enables storage and transportation of a preparation or the like containing a virus at both a refrigerated temperature (2 to 8°C) and a room temperature of about 25°C without decreasing viral capabilities such as infectivity. Development of such materials is desired.

In view of the above circumstances, an object of the present invention is to provide a virus stabilizer which enables storage and transportation of a preparation or the like containing a virus without the necessity of a freezer or the like by suppressing a decrease in viral capabilities at both a refrigerated temperature (2 to 8°C) and a room temperature of about 25°C, a gelatin hydrolysate for a virus stabilizer, and a virus-containing composition.

### SOLUTION TO PROBLEM

The present inventors have extensively conducted studies, and resultantly arrived at the present invention. That is, first, attention was paid to a gelatin hydrolysate obtained by hydrolyzing gelatin having an isoelectric point pH of about 5 to have a predetermined weight average molecular weight. Further, it has been found that when a virus is incorporated in a preparation containing the gelatin hydrolysate at a predetermined concentration, a decrease in viral capabilities such as infectivity can be suppressed at both a refrigerated temperature (2 to 8°C) and a room temperature of about 25°C, and hence, enabling stable storage and transportation of a preparation or the like containing a virus without the necessity of a freezer or the like. Based on these findings, the present invention has been completed.

The present invention has the following features.
[1] A virus stabilizer according to the present invention is a virus stabilizer comprising a gelatin hydrolysate and an aqueous medium, the virus stabilizer containing the gelatin hydrolysate at not less than 1 mass% and not more than 20 mass%, the gelatin hydrolysate having a weight average molecular weight of not more than 10000, the gelatin hydrolysate having an isoelectric point pH of not less than 4.0 and not more than 7.0.
[2] Preferably, the gelatin hydrolysate has a weight average molecular weight of not more than 6000.
[3] Preferably, the gelatin hydrolysate is a hydrolysate of alkali-treated gelatin.
[4] Preferably, the virus stabilizer contains the gelatin hydrolysate at more than 2 mass% and not more than 10 mass%.
[5] Preferably, the aqueous medium contains a salt having a buffering action.
[6] Preferably, the aqueous medium contains at least one saccharide selected from the group consisting of sucrose, lactose, sorbitol, inositol, trehalose, mannitol, maltitol, xylitol, erythritol and glycerol.
[7] Preferably, the aqueous medium contains at least one amino acid selected from the group consisting of methionine, arginine, tryptophane, glutamine and glutamic acid.
[8] A gelatin hydrolysate for a virus stabilizer according to the present invention has a weight average molecular weight of not more than 10000, and an isoelectric point pH of not less than 4.0 and not more than 7.0.
[9] Preferably, the gelatin hydrolysate for a virus stabilizer is liquid or powder.
[10] A virus-containing composition according to the present invention comprises the virus stabilizer and a virus.
[11] Preferably, the virus comprises at least one selected from the group consisting of a DNA virus having an envelope, a DNA virus having no envelope, an RNA virus having an envelope, and an RNA virus having no envelope.
[12] The virus is preferably a transgenic virus.
[13] In the virus-containing composition, a difference between the logarithmic reduction value of a virus titer in the case of storage at 25°C for 21 days and the logarithmic reduction value of a virus titer in the case of storage at -80°C for 21 days is preferably not more than 1.5 log.
[14] In the virus-containing composition, a difference between the logarithmic reduction value of a virus titer in the case of storage at 4°C for 210 days and the logarithmic reduction value of a virus titer in the case of storage at -80°C for 210 days is preferably not more than 1.0 log.
[15] In the virus-containing composition, the logarithmic reduction value when freezing and thawing is repeated three times is preferably not more than 0.3 log.

### ADVANTAGEOUS EFFECTS OF INVENTION

According to the above, it is possible to provide a virus stabilizer which enables storage and transportation of a preparation or the like containing a virus without the necessity of a freezer or the like by suppressing a decrease in viral capabilities at both a refrigerated temperature (2 to 8°C) and a room temperature of about 25°C, a gelatin hydrolysate for a virus stabilizer, and a virus-containing composition.

### DESCRIPTION OF EMBODIMENTS

Hereinafter, embodiments according to the present invention (hereinafter, also referred to as "the present embodiment") will be described in more detail. Here, as used herein, the writing form "A to B" means the upper limit and the lower limit of a range (i.e. not less than A and not more than B), and when a unit is not set forth for A, but is set forth only for B, the unit for A is identical to the unit for B. As used herein, the term "virus titer" refers to a minimum concentration at which a virus in a specimen ("virus-containing composition" as used herein) can infect a cell, i.e. a maximum dilution factor. Further, the phrase "the virus titer "decreases"" means that the dilution factor decreases, i.e. the minimum concentration at which the virus can infect a cell rises. As used herein, the term "gelatin" may be used when any of a substance name, a gelatin gel and a gelatin solution is mentioned. As in the case of the gelatin, the term "gelatin hydrolysate" may be used when a solution of a gelatin hydrolysate is mentioned.

### [Virus stabilizer]

The virus stabilizer according to the present embodiment is a virus stabilizer comprising a gelatin hydrolysate and an aqueous medium. The virus stabilizer contains the gelatin hydrolysate at not less than 1 mass% and not more than 20 mass%. The gelatin hydrolysate has a weight average molecular weight of not more than 10000. The gelatin hydrolysate has an isoelectric point pH of not less than 4.0 and not more than 7.0. Due to having these characteristics, the virus stabilizer according to the present embodiment can suppress a decrease in virus titer at both a refrigerated temperature (2 to 8°C) and a room temperature of about 25°C, and hence, when a virus-containing composition containing the virus stabilizer and a virus is made, the virus-containing composition can be stably stored and transported without the necessity of a freezer or the like.

### <Gelatin hydrolysate>

The virus stabilizer contains a gelatin hydrolysate as described above. As used herein, the "gelatin hydrolysate" refers to an aggregate of peptides (hydrolysate) obtained by hydrolyzing both or one of gelatin and collagen. That is, the term "gelatin hydrolysate" means an equivalent to an aggregate of peptides which is generally referred to as a collagen peptide or a collagen hydrolysate. Above all, the gelatin hydrolysate contained in the virus stabilizer according to the present embodiment has the above-described weight average molecular weight and isoelectric point pH. Further, the gelatin hydrolysate means an aggregate of peptides as described above, and therefore has the same characteristics as collagen and gelatin, such as having a primary structure in which glycine is repeated every three residues in an amino acid sequence constituting a peptide chain, etc.

As used herein, the term "gelatin" means a polypeptide in which a triple-helical structure of collagen has been uncoils by thermal denaturation, acid denaturation or other means, a chemically modified product thereof, and a pharmaceutically acceptable salt thereof. Specifically, the gelatin can be obtained by performing a conventionally known treatment such as a defatting treatment, a decalcification treatment, an acid or alkali treatment or a hot water extraction treatment on collagen derived from at least one selected from the group consisting of the following first to sixth groups. The gelatin may be a polypeptide obtained by a fermentation method using a microorganism, a recombinant polypeptide obtained by chemical synthesis or gene recombination, or a synthesized polypeptide. The term "collagen" refers to a protein derived from an extracellular matrix in any of skins and the like of vertebrate animals, which are classified into the following first to sixth groups. The collagen has a right-handed helical structure consisting of three peptide chains, and amino acid residues constituting the peptide chains have a primary structure in which a glycine residue is repeated every three residues (a so-called collagen-like sequence).
First group: group consisting of hide, skin, bone, cartilage and tendon of cattle.
Second group: group consisting of hide, skin, bone, cartilage and tendon of pig.
Third group: group consisting of hide, skin, bone, cartilage and tendon of sheep.
Fourth group: group consisting of hide, skin, bone, cartilage and tendon of chicken.
Fifth group: group consisting of hide, skin, bone, cartilage and tendon of ostrich.
Sixth group: group consisting of bone, hide and scale of fish.

Here, the term "chemically modified product" of the polypeptide (gelatin) means a polypeptide in which an amino group, a carboxyl group, a hydroxyl group, a thiol group or other group in amino acid residues constituting gelatin is chemically modified. The chemically modified gelatin can have a different solubility in water, isoelectric point and the like. Specifically, chemical modification such as O-acetylation can be performed on the hydroxy group of a hydroxyproline residue in gelatin. Chemical modification such as esterification or amidation can be performed on the α-carboxyl group of a glycine residue in gelatin. Chemical modification such as polypeptidylation, succinylation, maleylation, acetylation, deamination, benzoylation, alkylsulfonation, allylsulfonation, dinitrophenylation, trinitrophenylation, carbamylation or thiolation can be performed on the α-amino group of a proline residue in gelatin.

As specific means and treatment conditions for chemical modification of gelatin, conventionally known chemical modification methods can be applied. For chemical modification of the hydroxy group of the hydroxyproline residue, for example, O-acetylation can be performed by, for example, applying acetic anhydride in an aqueous solvent or a nonaqueous solvent. For chemical modification of the α-carboxyl group of the glycine residue, for example, esterification can be performed by, for example, feeding dry hydrogen chloride gas after suspension in methanol. For chemical modification of the α-carboxyl group of the glycine residue, amidation can be performed by applying carbodiimide or the like.

Further, the "derivative" of the polypeptide (gelatin) may include gelatin derivatives obtained by introducing a functional group into gelatin, copolymers of gelatin with lactic acid, glycol or the like, and copolymers of gelatin with polyethylene glycol or propylene glycol. As the gelatin derivative, derivatives obtained by introducing a functional group such as a guanidine group, a thiol group, an amino group, a carboxyl group, a sulfate group, a phosphate group, an alkyl group, an acyl group, a phenyl group or a benzyl group into gelatin can be exemplified.

The term "pharmaceutically acceptable salt" of the polypeptide (gelatin) means a salt which is pharmaceutically acceptable and has the desired activity (e.g. gelation ability) of the original polypeptide (gelatin). As the pharmaceutically acceptable salt, for example, inorganic acid salts such as hydrochlorides, sulfates, phosphates and hydrobromides, organic acid salts such as acetates, methanesulfonates, benzenesulfonates, p-toluenesulfonates, succinates, oxalates, fumarates and maleates, inorganic base salts such as sodium salts, potassium salts and calcium salts, and organic base salts such as triethylammonium salts can be exemplified. A specific peptide in gelatin can be converted into a form of a pharmaceutically acceptable salt in accordance with a conventional method.

Gelatin is a polypeptide derived from collagen possessed by many organisms, and therefore has excellent biocompatibility. Thus, the gelatin hydrolysate obtained by hydrolyzing the collagen and gelatin also has excellent biocompatibility, and is suitable as a component of a virus stabilizer for medicinal use.

When dissolved in a solvent (e.g. water), the gelatin hydrolysate does not turn into a gel and maintains a sol form not only at a room temperature of about 25°C but also in an environment at 2 to 8°C. Therefore, the virus stabilizer does not require a heating operation for turning into a sol when used at a room temperature of about 25°C. In the present embodiment, the term "sol" means a dispersion system including a dispersed material and a dispersion medium, where the dispersion medium is liquid. The term "gel" means a dispersion system including a dispersed material and a dispersion medium, where the dispersed material forms a crosslinked structure and the dispersion system loses flowability as a whole.

The gelatin hydrolysate is obtained by hydrolyzing both or one of gelatin and collagen as described above. The "hydrolyzation" here includes all of hydrolyzation with an acid, hydrolyzation with a base, hydrolyzation with an enzyme, and hydrolyzation with heat. Preferably, the gelatin hydrolysate is obtained by hydrolyzation with heat from the viewpoint of preventing contamination with impurities. Further, when both or one of gelatin and collagen is hydrolyzed with an enzyme, examples of the enzyme include collagenase, thiol protease, serine protease, acidic protease, alkaline protease and metalloprotease. As the enzyme, the above-mentioned enzymes can be used alone, or in combination of two or more thereof. Examples of the thiol protease include chymopapain, papain, bromelain and ficin which are derived from plants, and cathepsin and calcium-dependent protease which are derived from animals. Examples of the serine protease include trypsin and cathepsin D. Examples of the acidic protease include pepsin and chymotrypsin.

As the enzyme used, enzymes other than those derived from pathogenic microorganisms (e.g. enzymes derived from non-pathogenic microorganisms) are preferable given that the gelatin hydrolysate is used for medicaments. Examples of the non-pathogenic microorganism from which the enzyme is derived include *Bacillus licheniforms, Bacillus subtillis, Aspergillus oryzae,* Streptomyces, and *Bacillus amyloliquefaciens.* As the enzyme, an enzyme derived from one of the above-described non-pathogenic microorganisms may be used, or enzymes derived from two or more of the above-described non-pathogenic microorganisms may be used in combination. As a specific method for treatment with an enzyme, a conventionally known method may be used.

When the "gelatin hydrolysate" is obtained from the gelatin by hydrolysis using one enzyme alone, the enzyme is preferably not collagenase. This is because if the gelatin is hydrolyzed using collagenase alone, a specific peptide in which the N-terminal of an amino acid sequence is glycine may be a main component (more than 50 mass%). The gelatin hydrolysate is an aggregate consisting of two or more peptides, and in the composition thereof, the amount of the specific peptide is less than 50 mass%.

The gelatin hydrolysate can be obtained as liquid by hydrolyzing both or one of gelatin and collagen by the above-described method, followed by purification. Further, the gelatin hydrolysate can be obtained as powder by heat-drying or freeze-drying the liquid by known means.

### (Concentration)

The virus stabilizer contains the gelatin hydrolysate at not less than 1 mass% and not more than 20 mass%. If the concentration of the gelatin hydrolysate contained in the virus stabilizer is less than 1 mass%, it is difficult to exhibit a desired effect because the action of suppressing a decrease in virus titer is limited when a virus-containing composition containing the virus stabilizer and a virus is formed. If the concentration of the gelatin hydrolysate contained in the virus stabilizer is more than 20 mass%, the ease of handling of the virus stabilizer may deteriorate. Preferably, the virus stabilizer contains the gelatin hydrolysate at more than 2 mass% and not more than 10 mass% from the viewpoint of further suppressing a decrease in virus titer. The concentration of the gelatin hydrolysate in the virus stabilizer can be measured by a known method, such as hydroxyproline quantitation.

### (Weight average molecular weight)

The gelatin hydrolysate has a weight average molecular weight of not more than 10000. Preferably, the gelatin hydrolysate has a weight average molecular weight of not more than 6000. More preferably, the gelatin hydrolysate has a weight average molecular weight of not more than 5000. This enables the virus stabilizer to maintain a sol form in an environment at about 2 to 30°C. Further, when a virus-containing composition containing a virus stabilizer and a virus is formed, the gelatin hydrolysate can suppress coagulation of the virus in the composition, so that it is possible to suppress a decrease in virus titer. While the detailed mechanism is unknown, it is considered that when the weight average molecular weight of the gelatin hydrolysate is in the above range, the gelatin hydrolysate can interact with a viral surface to a moderate degree regardless of the presence or absence of an envelope as both a hydrophilic region and a hydrophobic region in the molecule are exposed to the molecular surface side (outer side), so that the gelatin hydrolysate can provide protection by covering the viral surface.

If the weight average molecular weight of the gelatin hydrolysate exceeds 10000, the gelatin hydrolysate may turn into a gel at 2 to 8°C. The lower limit of the weight average molecular weight of the gelatin hydrolysate is not particularly limited, and is, for example, equal to the molecular weight of glycine, which is 75.

The weight average molecular weight of the gelatin hydrolysate can be determined by carrying out gel filtration chromatography under the following measurement conditions.
Equipment: High-performance liquid chromatograph (HPLC) (manufactured by TOSOH CORPORATION)
Column: TSKGel (registered trademark) G2000 SW_{XL}
Column temperature: 30°C
Eluent: Acetonitrile at 40 mass% (containing TFA at 0.05 mass%)
Flow rate: 0.5 mL/min
Injection amount: 10 µL
Detection: UV 220 nm
Molecular weight marker: The following three types are used:
Cytochrome C Mw: 12384
Aprotinin Mw: 6512
Bacitracin Mw: 1423

Specifically, substantially 0.5 g of a virus stabilizer containing the gelatin hydrolysate is added to about 100 ml of distilled water, and the mixture is stirred, and then filtered with a 0.2 µm filter to prepare a sample (object to be measured) for measuring the weight average molecular weight. By measuring the object to be measured on the basis of the above conditions for gel filtration chromatography, the weight average molecular weight of the gelatin hydrolysate can be determined.

### (Isoelectric point)

The gelatin hydrolysate has an isoelectric point pH of not less than 4.0 and not more than 7.0. The isoelectric point pH of the gelatin hydrolysate is preferably 4.0 to 5.5, more preferably not less than 4.0 and not more than 4.8. A gelatin hydrolysate having an isoelectric point pH in such a range can be efficiently obtained by hydrolyzing collagen subjected to an alkali treatment, or hydrolyzing gelatin obtained from collagen subjected to an alkali treatment (so-called alkali-treated gelatin). That is, the gelatin hydrolysate is preferably a hydrolysate of alkali-treated gelatin. In particular, the gelatin hydrolysate is preferably a hydrolysate of alkali-treated gelatin which has an isoelectric point pH of about 4.8 to 5.5. In general, gelatin obtained by treating collagen with an inorganic acid is referred to as acid-treated gelatin, and gelatin obtained by treating collagen with an inorganic base is referred to as alkali-treated gelatin. Specifically, the alkali-treated gelatin can be obtained by treating collagen with an inorganic base such as sodium hydroxide, calcium hydroxide or potassium hydroxide. The acid-treated gelatin has an isoelectric point pH of 8 to 9. On the other hand, the alkali-treated gelatin has an isoelectric point pH of 4.0 to 7.0. As such alkali-treated gelatin, for example, alkali-treated pig hide-derived gelatin (trade name: "beMatrix (registered trademark) Gelatin LS-H", manufactured by Nitta Gelatin Inc.) can be exemplified.

When a virus-containing composition is formed from a virus stabilizer containing a gelatin hydrolysate having an isoelectric point pH within the above-described range, and a virus, the gelatin hydrolysate can suppress coagulation of the virus, and hence, it is possible to suppress a decrease in virus titer in the composition. While the detailed mechanism is unknown, a gelatin hydrolysate having an isoelectric point pH within the above-mentioned range has negative charge in an amount relatively larger than that of positive charge in the molecule, and therefore is negatively charged as a whole, but has both a positively charged portion and a negatively charged portion. Thus, the gelatin hydrolysate can bind to the virus because of weak electrostatic interaction occurring between the positively charged portion and capsid protein, spike protein, envelope protein on a viral surface, and the like which are negatively charged in the virus. Meanwhile, the gelatin hydrolysate mutually repels other peptide chains forming the virus and the gelatin hydrolysate in the negatively charged portion. Thus, it is considered that the gelatin hydrolysate can protect the virus by covering the viral surface while suppressing coagulation of the virus. On the other hand, a gelatin hydrolysate obtained from acid-treated gelatin having an isoelectric point pH of about 9 (e.g., a gelatin hydrolysate disclosed in Patent Literature 1) has positive charge, and therefore may coagulate the virus by exhibiting strong electrostatic interaction with capsid protein etc. which is negatively charged. The gelatin hydrolysate obtained from the acid-treated gelatin may have a marked action of coagulating the virus, particularly in an environment at a temperature of about 25°C.

The isoelectric point pH of the gelatin hydrolysate can be determined by measuring the isoelectric point pH of both or one of gelatin and collagen as raw materials for the gelatin hydrolysate using a conventionally known method, and use of the following method for measuring an isoelectric point using a zeta potential as an index is preferable because the value of an isoelectric point can be more accurately determined. That is, first, a gelatin hydrolysate to be measured is dissolved in an acetate buffer solution (pH: 4.0 to 5.5) to obtain a 0.4 w/v% solution to be measured. Next, the solution to be measured is filtered with a 0.22 µm filter (manufactured by Merck KGaA), and a capillary cell is then filled with 0.8 mL of the solution to be measured in such a manner that air bubbles does not enter. Subsequently, the capillary cell filled with the solution to be measured is set in a zeta potential measuring apparatus (manufactured by Malvern Panalytical Ltd) to measure the zeta potential at each pH at 25°C. Here, the pH value at which the zeta potential is 0 can be determined as an isoelectric point of the solution to be measured (gelatin hydrolysate to be measured).

### <Aqueous medium>

The virus stabilizer contains an aqueous medium as described above. As used herein, the term "aqueous medium" refers to a medium in which the gelatin hydrolysate is dissolved or dispersed, where the medium may contain components other than water, such as amino acids, saccharides and salts having a buffering action as described later. For example, the aqueous medium may be a buffer solution containing a salt having a buffering action. Specifically, the aqueous medium may be a GTS buffer. That is, it is preferable that the aqueous medium contain a salt having a buffering action. This enables the virus stabilizer to contribute to stabilization of a virus by the buffering action when a virus-containing composition is formed by incorporating the virus.

### (Salt having buffering action)

As the salt having a buffering action, sodium phosphate, potassium phosphate, calcium phosphate, magnesium phosphate, sodium hydrogen phosphate, potassium hydrogen phosphate, calcium hydrogen phosphate, magnesium hydrogen phosphate, sodium chloride, potassium chloride and the like can be exemplified. The aqueous medium may contain one of the salts having a buffering action, or contain two or more thereof in combination. As the aqueous medium containing a salt having a buffering action, the GTS buffer, a PBS buffer, a Tris buffer, a HEPES buffer, a citrate buffer and the like can be exemplified. The composition of the GTS buffer can be, for example, 2.5 mass% glycerol, 20 mM Tris (pH 8) and 25 mM NaCl.

### (Amino acid)

Preferably, the aqueous medium contains at least one amino acid selected from the group consisting of methionine, arginine, tryptophane, glutamine and glutamic acid. The aqueous medium may contain one selected from the group of these amino acids, or contain two or more of the amino acids in combination. More preferably, the aqueous medium contains both or one of the amino acids of methionine and arginine. This enables the virus stabilizer to further contribute to stabilization of a virus when a virus-containing composition is formed by incorporating the virus.

### (Saccharide)

Preferably, the aqueous medium contains at least one saccharide selected from the group consisting of sucrose, lactose, sorbitol, inositol, trehalose, mannitol, maltitol, xylitol, erythritol and glycerol. The aqueous medium may contain one selected from the group of saccharides, or contain two or more of the saccharides in combination. More preferably, the aqueous medium contains at least one of sucrose, lactose and sorbitol. This enables the virus stabilizer to further contribute to stabilization of a virus when a virus-containing composition is formed by incorporating the virus. The compound contained in the "saccharide" herein includes not only organic compounds classified generally as saccharides, but also organic compounds classified as sugar alcohols. The organic compounds classified as sugar alcohols in the above-described group of saccharides include the sorbitol, mannitol, maltitol, xylitol, erythritol and glycerol described above.

### (Other components)

The aqueous medium may contain other components as long as the effects of the present invention are exhibited. Examples of the other components include growth factors, differentiation factors, hormones, chemokines, cytokines, cell adhesion molecules, chemotactic factors, enzymes, enzyme inhibitors, coenzymes (vitamins), minerals, fats, lipids, stabilizing agents and preservatives.

### <Effect>

The virus stabilizer according to the present embodiment comprises an aqueous medium as described above, and a gelatin hydrolysate contained at a concentration of not less than 1 mass% and not more than 20 mass%. The gelatin hydrolysate has a weight average molecular weight of not more than 10000 and an isoelectric point pH of not less than 4.0 and not more than 7.0. When a virus-containing composition as described later is formed by incorporating a virus in the virus stabilizer according to the present embodiment, coagulation of the virus in the virus-containing composition can be suppressed to prevent a detrimental effect on the infectivity and the like of the virus. Hence, a decrease in virus titer can be suppressed, so that the virus-containing composition can be stably stored and transported at both a refrigerated temperature (2 to 8°C) and a room temperature of about 25°C.

### [Gelatin hydrolysate for virus stabilizer]

The gelatin hydrolysate for a virus stabilizer according to the present embodiment has a weight average molecular weight of not more than 10000 and an isoelectric point pH of not less than 4.0 and not more than 7.0. Preferably, the gelatin hydrolysate for a virus stabilizer according to the present embodiment has a weight average molecular weight of not more than 6000 and an isoelectric point pH of 4.0 to 5.5. Specifically, the gelatin hydrolysate for a virus stabilizer has characteristics as described in the section <Gelatin hydrolysate> as a gelatin hydrolysate, and the descriptions of these characteristics are not repeated. The gelatin hydrolysate for a virus stabilizer has, as gelatin hydrolysate's attribute that has not been heretofore known, i.e., an unknown attribute, an action such that when a virus-containing composition as described later is formed by incorporating a virus, coagulation of the virus in the virus-containing composition is suppressed. Hence, the gelatin hydrolysate for a virus stabilizer can be provided for use in the virus stabilizer, and the virus-containing composition can be stably stored and transported without the necessity of a freezer or the like at both a refrigerated temperature (2 to 8°C) and a room temperature of about 25°C.

The gelatin hydrolysate for a virus stabilizer is preferably liquid or powder. This enables a virus-containing composition to be easily prepared by adding a virus together with an aqueous medium when the gelatin hydrolysate for a virus stabilizer is liquid. When the gelatin hydrolysate for a virus stabilizer is powder, a virus-containing composition can be easily prepared by dissolving or dispersing the powder in an aqueous medium to prepare a virus stabilizer, and adding a virus thereto. The gelatin hydrolysate for a virus stabilizer can be prepared as liquid by hydrolyzing both or one of gelatin and collagen, and then performing purification. The gelatin hydrolysate for a virus stabilizer can be prepared as powder by heat-drying or freeze-drying the thus-prepared liquid gelatin hydrolysate for a virus stabilizer by a known method.

### [Virus-containing composition]

The virus-containing composition according to the present embodiment contains the virus stabilizer and a virus. Since the virus-containing composition contains the virus stabilizer, coagulation of the virus in the composition can be suppressed, so that it is possible to suppress a decrease in virus titer. Hence, the virus-containing composition can be stably stored and transported without the necessity of a freezer or the like at both a refrigerated temperature (2 to 8°C) and a room temperature of about 25°C. Here, specifically, the virus stabilizer contained in the virus-containing composition has characteristics as described in the section [Virus stabilizer], and the descriptions of these characteristics are not repeated.

### <Virus>

The virus-containing composition contains a virus as described above. Preferably, the virus comprises at least one selected from the group consisting of a DNA virus having an envelope, a DNA virus having no envelope, an RNA virus having an envelope, and an RNA virus having no envelope. That is, the type of a virus forming the virus-containing composition does not limit the use of the virus. Further, the virus-containing composition may contain one selected from the above-described group of viruses, or contain two or more of the viruses in combination. Even in this case, the virus-containing composition enables the relevant virus to be stably stored and transported at both a refrigerated temperature (2 to 8°C) and a room temperature of about 25°C because the virus-containing composition contains the virus stabilizer.

As the virus, specifically, viruses that are used for live vaccine or inactivated vaccine, such as influenza viruses and rotaviruses, oncolytic viruses having an action of lysing tumors, simple herpes viruses, vaccinia viruses, adenoviruses, coxsackieviruses, poxviruses, paramyxoviruses, picornaviruses, rhabdoviruses, reoviruses, parvoviruses, bracken viruses, orthomyxoviruses, retroviruses and the like can be exemplified. Further, the virus may be a transgenic virus.

### <Other components>

The virus-containing composition may contain other components as long as the effects of the present invention are exhibited. Examples of the other components include growth factors, differentiation factors, hormones, chemokines, cytokines, cell adhesion molecules, chemotactic factors, enzymes, enzyme inhibitors, coenzymes (vitamins), minerals, fats, lipids, stabilizing agents and preservatives.

### <Logarithmic reduction value (LRV) of virus titer>

In the virus-containing composition, a difference between the logarithmic reduction value (hereinafter, also referred to as "LRV") of a virus titer in the case of storage at 25°C for 21 days and LRV of a virus titer in the case of storage at -80°C for 21 days is preferably not more than 1.5 log. The difference is more preferably not more than 1.0 log. In the virus-containing composition, the lower limit value of the difference is preferably 0.

In the virus-containing composition, a difference between LRV of a virus titer in the case of storage at 4°C for 210 days and LRV of a virus titer in the case of storage at -80°C for 210 days is preferably not more than 1.0 log. The difference is more preferably not more than 0.5 log. In the virus-containing composition, the lower limit value of the difference is preferably 0.

The virus-containing composition enables a virus to be stably stored and transported at both a refrigerated temperature (2 to 8°C) and a room temperature of about 25°C when having the above-described characteristics for LRV of the virus titer.

Further, in the virus-containing composition, it is also preferable that a difference between LRV of a virus titer in the case of storage at 25°C for 21 days and LRV of a virus titer in the case of storage at -80°C for 21 days be not more than 1.5 log and a difference between LRV of a virus titer in the case of storage at 4°C for 21 days and LRV of a virus titer in the case of storage at -80°C for 21 days be not more than 1.0 log.

In the virus-containing composition, the logarithmic reduction value of a virus titer when freezing and thawing is repeated three times is preferably not more than 0.3 log. This enables the virus-containing composition to maintain the virus titer even when freezing and thawing is repeatedly performed up to three times. Hence, the virus-containing composition can be used flexibly, which is extremely convenient.

### (Method for measuring logarithmic reduction value (LRV) of virus titer)

LRV of a virus titer in the virus-containing composition can be determined using a conventional method for measuring a virus titer. For example, the LRV can be obtained by determining TCID 50 of a virus in the virus-containing composition. The term "TCID 50" refers to a degree of dilution of a virus at which when a virus dilution liquid (herein, a dilution liquid of a "virus-containing composition) is inoculated in a test tube or on a culture dish such as a 96-well flat-bottom plate in which cells are cultured and deposited beforehand, infection of 50% of the cells is confirmed. The infection can be confirmed with a microscope using, for example, a cytopathic effect as an index. LRV of a virus titer can be calculated on the basis of TCID 50 following the calculation expression of the Beherens-Karber method.

The method for measuring LRV of a virus titer can be as follows. Specifically, first, DMEM culture medium can be used as a dilution liquid for dilution of a virus-containing composition stored for a predetermined period. Cell culture can be performed by seeding a predetermined cell suspension liquid on a 96-well flat-bottom plate (manufactured by TPP Company) at 100 µL/well (about 10⁴/well), and incubating the cell suspension liquid overnight with a CO₂ incubator (CO₂ concentration: 5%) at 37°C.

Thereafter, the virus-containing composition is stepwise diluted by a factor of 10 with DMEM culture medium. The diluted virus is added to predetermined cells (various cells corresponding to the type of a virus contained in the virus-containing composition (e.g., LCC-MK 2 cells, MDBK cells or HEK 293 cells)) in the 96-well flat-bottom plate at 50 µL/well, and cultured with a COz incubator (CO₂ concentration: 5%) at 37°C. On day 7, the virus titer (TCID 50) is determined by using CPE as an index and following the above-described calculation expression. On the basis of the TCID 50 value, LRV can be calculated.

When the type of a virus contained in the virus-containing composition is rNDV-GFP, LRV of a virus titer can be calculated as follows. That is, the diluted virus is added to Vero cells in a 24-well flat-bottom plate at 50 µL/well, and cultured for 24 to 48 hours with a CO₂ incubator (CO₂ concentration: 5%) at 37°C, and the cells are harvested with 0.25 mass% of trypsin EDTA (manufactured by Sigma-Aldrich Co. LLC). Thereafter, the cells are washed with DMEM culture medium and PBS. Further, the cells are suspended in PBS containing 0.5 mass% formalin, the number of infected cells is measured by FCM analysis or a fluorescent microscope, and the above-described calculation expression is followed to determine the virus titer (FFU/mL). Thereafter, on the basis of the virus titer (FFU/mL), LRV can be calculated.

### <Method for preparing virus stabilizer>

The virus stabilizer according to the present embodiment can be obtained preferably by the following method. That is, the virus stabilizer can be obtained through the step of preparing a gelatin hydrolysate (first step) and the step of preparing a virus stabilizer comprising the gelatin hydrolysate and an aqueous medium (second step).

### (First step)

The first step is a step of preparing a gelatin hydrolysate. The gelatin hydrolysate can be prepared by hydrolyzing both or one of gelatin having an isoelectric point pH of not less than 4.0 and not more than 7.0 and collagen made to have an isoelectric point pH of not less than 4.0 and not more than 7.0 to a weight average molecular weight of not more than 10000 as described above. It is preferable to use alkali-treated gelatin as the gelatin having an isoelectric point pH of not less than 4.0 and not more than 7.0. It is also possible to provide a gelatin hydrolysate from a commercially available product (e.g., trade name: "beMatrix (registered trademark) Gelatin LS-H", manufactured by Nitta Gelatin Inc.).

### (Second step)

The second step is a step of preparing a virus stabilizer from the gelatin hydrolysate and an aqueous medium. The virus stabilizer can be prepared by mixing the gelatin hydrolysate and the aqueous medium at a mass ratio such that the concentration of the gelatin hydrolysate is not less than 1 mass% and not more than 20 mass%. As a specific mixing method, a conventionally known method can be used. The aqueous medium can be prepared by a conventionally known method such as a method in which a salt having a buffering action is added to ion-exchanged water to a predetermined concentration.

### <Method for preparing virus-containing composition>

The virus-containing composition can be prepared by adding a virus to the virus stabilizer. The virus can be added to the virus stabilizer by a conventionally known method. The virus can be added to the virus stabilizer so that normally, the virus titer (TCID 50) is ultimately not less than 10⁵/mL. As above, the virus-containing composition according to the present embodiment can be obtained.

### Examples

Hereinafter, the present invention will be described in more detail by way of Examples, which should not be construed as limiting the present invention.

### [Preparation of sample]

The samples (samples 1 to 17) of virus-containing compositions for use in various experiments (experiments 1 to 6) described below were provided in the manner described below. Samples 1 to 4, samples 9 to 12 and samples 14 to 17 correspond to Examples, and samples 5 to 8 and sample 13 correspond to Comparative Examples.

### <Sample 1>

Alkali-treated pig hide-derived gelatin "beMatrix (registered trademark) Gelatin LS-H (manufactured by Nitta Gelatin Inc.)" having an isoelectric point pH of 5 was hydrolyzed by heat to a weight average molecular weight of 3800 to obtain a gelatin hydrolysate. Subsequently, the gelatin hydrolysate was dissolved in a GTS buffer as an aqueous medium so as to achieve a content of 5 mass% to obtain a virus stabilizer. To the virus stabilizer, a bovine herpesvirus (BHV-1) which is a DNA virus having an envelope was added to a TCID 50 of 2 × 10^{8.1}/mL to obtain a virus-containing composition. Further, the virus-containing composition was divided into seven specimens, which were stored, respectively, for 21 days, 56 days and 210 days at 4°C, for 21 days at 25°C, and for 21 days, 56 days and 210 days at -80°C.

### <Sample 2>

Alkali-treated pig hide-derived gelatin "beMatrix (registered trademark) Gelatin LS-H (manufactured by Nitta Gelatin Inc.)" having an isoelectric point pH of 5 was hydrolyzed by heat to a weight average molecular weight of 650 to obtain a gelatin hydrolysate. Subsequently, the gelatin hydrolysate was dissolved in the GTS buffer so as to achieve a content of 5 mass% to obtain a virus stabilizer. Thereafter, a virus was added to the virus stabilizer by a method identical to that for sample 1 to obtain a virus-containing composition. The virus-containing composition was divided into four specimens, which were stored, respectively, for 56 days at 4°C, for 21 days at 25°C, and for 21 days and 56 days at -80°C.

### <Sample 3>

Alkali-treated pig hide-derived gelatin "beMatrix (registered trademark) Gelatin LS-H (manufactured by Nitta Gelatin Inc.)" having an isoelectric point pH of 5 was hydrolyzed by heat to a weight average molecular weight of 1980 to obtain a gelatin hydrolysate. Subsequently, the gelatin hydrolysate was dissolved in the GTS buffer so as to achieve a content of 5 mass% to obtain a virus stabilizer. Thereafter, a virus was added to the virus stabilizer by a method identical to that for sample 1 to obtain a virus-containing composition. The virus-containing composition was divided into four specimens, which were stored, respectively, for 56 days at 4°C, for 21 days at 25°C, and for 21 days and 56 days at -80°C.

### <Sample 4>

Alkali-treated pig hide-derived gelatin "beMatrix (registered trademark) Gelatin LS-H (manufactured by Nitta Gelatin Inc.)" having an isoelectric point pH of 5 was hydrolyzed by heat to a weight average molecular weight of 9890 to obtain a gelatin hydrolysate. Subsequently, the gelatin hydrolysate was dissolved in the GTS buffer so as to achieve a content of 5 mass% to obtain a virus stabilizer. Thereafter, a virus was added to the virus stabilizer by a method identical to that for sample 1 to obtain a virus-containing composition. The virus-containing composition was divided into four specimens, which were stored, respectively, for 56 days at 4°C, for 21 days at 25°C, and for 21 days and 56 days at -80°C.

### <Sample 5>

Alkali-treated pig hide-derived gelatin "beMatrix (registered trademark) Gelatin LS-H (manufactured by Nitta Gelatin Inc.)" having an isoelectric point pH of 5 was hydrolyzed by heat to a weight average molecular weight of 59800 to obtain a gelatin hydrolysate. Subsequently, the gelatin hydrolysate was dissolved in the GTS buffer so as to achieve a content of 5 mass% to obtain a virus stabilizer. Thereafter, a virus was added to the virus stabilizer by a method identical to that for sample 1 to obtain a virus-containing composition. Further, the virus-containing composition was divided into four specimens, which were stored, respectively, for 56 days at 4°C, for 21 days at 25°C, and for 21 days and 56 days at -80°C.

### <Sample 6>

A gelatin hydrolysate obtained from acid-treated pig hide-derived gelatin having an isoelectric point pH of 9 (manufactured by Nitta Gelatin Inc.) was hydrolyzed by heat to a weight average molecular weight of 2060 to obtain a gelatin hydrolysate. Subsequently, the gelatin hydrolysate was dissolved in the GTS buffer so as to achieve a content of 5 mass% to obtain a virus stabilizer. Thereafter, a virus was added to the virus stabilizer by a method identical to that for sample 1 to obtain a virus-containing composition. The virus-containing composition was divided into four specimens, which were stored, respectively, for 56 days at 4°C, for 21 days at 25°C, and for 21 days and 56 days at -80°C.

### <Sample 7>

Alkali-treated pig hide-derived gelatin "beMatrix (registered trademark) Gelatin LS-H (manufactured by Nitta Gelatin Inc.)" having an isoelectric point pH of 5 was hydrolyzed by heat to a weight average molecular weight of 3800 to obtain a gelatin hydrolysate. Subsequently, a virus-containing composition was obtained by a method identical to that for sample 1 except that the gelatin hydrolysate was dissolved in the GTS buffer so as to achieve a content of 0.1 mass% to obtain a virus stabilizer. The virus-containing composition was divided into four specimens, which were stored, respectively, for 56 days at 4°C, for 21 days at 25°C, and for 21 days and 56 days at - 80°C.

### <Sample 8>

Alkali-treated pig hide-derived gelatin "beMatrix (registered trademark) Gelatin LS-H (manufactured by Nitta Gelatin Inc.)" having an isoelectric point pH of 5 was hydrolyzed by heat to a weight average molecular weight of 3800 to obtain a gelatin hydrolysate. Subsequently, a virus-containing composition was obtained by a method identical to that for sample 1 except that the gelatin hydrolysate was dissolved in the GTS buffer so as to achieve a content of 0.5 mass% to obtain a virus stabilizer. The virus-containing composition was divided into four specimens, which were stored, respectively, for 56 days at 4°C, for 21 days at 25°C, and for 21 days and 56 days at - 80°C.

### <Sample 9>

Alkali-treated pig hide-derived gelatin "beMatrix (registered trademark) Gelatin LS-H (manufactured by Nitta Gelatin Inc.)" having an isoelectric point pH of 5 was hydrolyzed by heat to a weight average molecular weight of 3800 to obtain a gelatin hydrolysate. Subsequently, a virus-containing composition was obtained by a method identical to that for sample 1 except that the gelatin hydrolysate was dissolved in the GTS buffer so as to achieve a content of 1 mass% to obtain a virus stabilizer. The virus-containing composition was divided into four specimens, which were stored, respectively, for 56 days at 4°C, for 21 days at 25°C, and for 21 days and 56 days at - 80°C.

### <Sample 10>

Alkali-treated pig hide-derived gelatin "beMatrix (registered trademark) Gelatin LS-H (manufactured by Nitta Gelatin Inc.)" having an isoelectric point pH of 5 was hydrolyzed by heat to a weight average molecular weight of 3800 to obtain a gelatin hydrolysate. Subsequently, a virus-containing composition was obtained by a method identical to that for sample 1 except that the gelatin hydrolysate was dissolved in the GTS buffer so as to achieve a content of 2.5 mass% to obtain a virus stabilizer. The virus-containing composition was divided into four specimens, which were stored, respectively, for 56 days at 4°C, for 21 days at 25°C, and for 21 days and 56 days at - 80°C.

### <Sample 11>

Alkali-treated pig hide-derived gelatin "beMatrix (registered trademark) Gelatin LS-H (manufactured by Nitta Gelatin Inc.)" having an isoelectric point pH of 5 was hydrolyzed by heat to a weight average molecular weight of 3800 to obtain a gelatin hydrolysate. Subsequently, a virus-containing composition was obtained by a method identical to that for sample 1 except that the gelatin hydrolysate was dissolved in the GTS buffer so as to achieve a content of 10 mass% to obtain a virus stabilizer. The virus-containing composition was divided into four specimens, which were stored, respectively, for 56 days at 4°C, for 21 days at 25°C, and for 21 days and 56 days at - 80°C.

### <Sample 12>

Alkali-treated pig hide-derived gelatin "beMatrix (registered trademark) Gelatin LS-H (manufactured by Nitta Gelatin Inc.)" having an isoelectric point pH of 5 was hydrolyzed by heat to a weight average molecular weight of 3800 to obtain a gelatin hydrolysate. Subsequently, a virus-containing composition was obtained by a method identical to that for sample 1 except that the gelatin hydrolysate was dissolved in the GTS buffer so as to achieve a content of 20 mass% to obtain a virus stabilizer. The virus-containing composition was divided into four specimens, which were stored, respectively, for 56 days at 4°C, for 21 days at 25°C, and for 21 days and 56 days at - 80°C.

### <Sample 13>

Recombinant human albumin "Recombumin (registered trademark) Elite (manufactured by Albumedix Ltd)" was dissolved in the GTS buffer so as to achieve a content of 1 mass% to obtain a virus stabilizer. Thereafter, a virus was added to the virus stabilizer by a method identical to that for sample 1 to obtain a virus-containing composition. Further, the virus-containing composition was divided into four specimens, which were stored, respectively, for 56 days at 4°C, for 21 days at 25°C, and for 21 days and 56 days at -80°C. Sample 13 is a heretofore known virus stabilizer.

### <Sample 14>

A virus-containing composition was obtained by a method identical to that for sample 1 except that a parainfluenza virus (PI-3) which is an RNA virus having an envelope was added to a virus stabilizer so as to achieve a TCID 50 of 2 × 10^{5.5}/mL. The virus-containing composition was divided into seven specimens, which were stored, respectively, for 21 days, 56 days and 210 days at 4°C, for 21 days at 25°C, and for 21 days, 56 days and 210 days at -80°C.

### <Sample 15>

A virus-containing composition was obtained by a method identical to that for sample 1 except that a reovirus (Reo-3) which is a RNA virus having no envelope was added to a virus stabilizer so as to achieve a TCID 50 of 2 × 10^{6.5}/mL. The virus-containing composition was divided into seven specimens, which were stored, respectively, for 21 days, 56 days and 210 days at 4°C, for 21 days at 25°C, and for 21 days, 56 days and 210 days at -80°C.

### <Sample 16>

A virus-containing composition was obtained by a method identical to that for sample 1 except that an adenovirus (Ad5) which is an RNA virus having no envelope was added to a virus stabilizer so as to achieve a TCID 50 of 2 × 10⁸/mL. The virus-containing composition was divided into seven specimens, which were stored, respectively, for 21 days, 56 days and 210 days at 4°C, for 21 days at 25°C, and for 21 days, 56 days and 210 days at -80°C.

### <Sample 17>

A virus-containing composition was obtained by a method identical to that for sample 1 except that an oncolytic virus paramyxovirus transgenic for GFP, where the virus is a paramyxovirus (Newcastle disease virus: rNDV-GFP) which is a RNA virus having an envelope, was added to a virus stabilizer so as to achieve a FFU content of 4 × 10⁶/mL. The virus-containing composition was divided into four specimens, which were stored, respectively, for 56 days at 4°C, for 21 days at 25°C, and for 21 days and 56 days at -80°C. Table 1 shows a listing of samples 1 to 17. Here, as the isoelectric point pH of the gelatin hydrolysate contained in the virus stabilizer of each of samples 1 to 4, samples 9 to 12 and samples 14 to 17, a value determined by the above-described method for measuring an isoelectric point using a zeta potential as an index is shown.

### [Table 1]

**Table 1**

| | Gelatin hydrolysate | | | | Buffer solution | Type of virus |
|---|---|---|---|---|---|---|
| | Concentration (mass%) | Weight average molecular weight (Mw) | Isoelectric point | Type of animal | | |
| Sample 1 | 5 | 3800 | 4.7 | Pig | GTS | BHV-1 |
| Sample 2 | 5 | 650 | 4.0 | Pig | GTS | BHV-1 |
| Sample 3 | 5 | 1980 | 4.3 | Pig | GTS | BHV-1 |
| Sample 4 | 5 | 9890 | 4.8 | Pig | GTS | BHV-1 |
| Sample 5 | 5 | 59800 | 5.0 | Pig | GTS | BHV-1 |
| Sample 6 | 5 | 2060 | 8.0 | Pig | GTS | BHV-1 |
| Sample 7 | 0.1 | 3800 | 4.7 | Pig | GTS | BHV-1 |
| Sample 8 | 0.5 | 3800 | 4.7 | Pig | GTS | BHV-1 |
| Sample 9 | 1 | 3800 | 4.7 | Pig | GTS | BHV-1 |
| Sample 10 | 2.5 | 3800 | 4.7 | Pig | GTS | BHV-1 |
| Sample 11 | 10 | 3800 | 4.7 | Pig | GTS | BHV-1 |
| Sample 12 | 20 | 3800 | 4.7 | Pig | GTS | BHV-1 |
| Sample 13 | 1 | - | - | Recombinant human | GTS | BHV-1 |
| Sample 14 | 5 | 3800 | 4.7 | Pig | GTS | PI-3 |
| Sample 15 | 5 | 3800 | 4.7 | Pig | GTS | Reo-3 |
| Sample 16 | 5 | 3800 | 4.7 | Pig | GTS | Ad5 |
| Sample 17 | 5 | 3800 | 4.7 | Pig | GTS | rNDV-GFP |

| | | | | | | |
|---|---|---|---|---|---|---|
| * For sample 13, the virus stabilizer was formed from albumin and an aqueous medium. | | | | | | |

### [Experiment 1: Study on suitable weight average molecular weight of gelatin hydrolysate]

For samples 1, 2, 3, 4 and 5, TCID 50 of each of the specimens stored for 56 days at 4°C and for 21 days at 25°C was determined, and on the basis of the relevant value of TCID 50, LRV of a virus titer at 4°C and LRV of a virus titer at 25°C were calculated. Further, for samples 1, 2, 3, 4 and 5, TCID 50 of each of the specimens stored for 21 days and 56 days at -80°C was determined, and on the basis of the relevant value of TCID 50, LRV of a virus titer at -80°C was calculated. Based on these, a difference between LRV of a virus titer in each specimen stored for 21 days at 25°C and LRV of a virus titer in each specimen stored for 21 days at -80°C was calculated for samples 1, 2, 3, 4 and 5. Table 2 shows the results. Further, Table 3 shows the results of calculating a difference between LRV of a virus titer in each specimen stored for 56 days at 4°C and LRV of a virus titer in each specimen stored for 56 days at -80°C for samples 1, 2, 3, 4 and 5.

### [Table 2]

**Table 2**

| | Weight average molecular weight of gelatin hydrolysate (Mw) | Logarithmic reduction value (LRV) difference between LRV at 25°C and LRV at -80°C (3W) |
|---|---|---|
| Sample 1 | 3800 | 0.7 |
| Sample 2 | 650 | 0.7 |
| Sample 3 | 1980 | 1 |
| Sample 4 | 9890 | 0.9 |
| Sample 5 | 59800 | 1.8 |

### [Table 3]

**Table 3**

| | Weight average molecular weight of gelatin hydrolysate (Mw) | Logarithmic reduction value (LRV) difference between LRV at 4°C and LRV at -80°C (8W) |
|---|---|---|
| Sample 1 | 3800 | 0.3 |
| Sample 2 | 650 | 0.4 |
| Sample 3 | 1980 | 0.5 |
| Sample 4 | 9890 | 0.5 |
| Sample 5 | 59800 | - |

From Tables 2 and 3 above, it can be evaluated that samples 1, 2, 3 and 4 enable a virus to be stably stored and transported at 4°C (refrigerated temperature) and 25°C (room temperature) because the LRV differences are not more than 1.0 log and not more than 1.5 log, respectively. In sample 5, the LRV difference exceeded 1.5 log at 25°C due to gelation. For sample 5, evaluation after storage for 56 days at 4°C was not performed because of the gelation.

### [Experiment 2: Study on suitable isoelectric point of gelatin hydrolysate]

For samples 1, 2, 3, 4 and 6, TCID 50 of each of the specimens stored for 56 days at 4°C and for 21 days at 25°C was determined, and on the basis of the relevant value of TCID 50, LRV of a virus titer at 4°C and LRV of a virus titer at 25°C were calculated. Further, for samples 1, 2, 3, 4 and 6, TCID 50 of each of the specimens stored for 21 days and 56 days at -80°C was determined, and on the basis of the relevant value of TCID 50, LRV of a virus titer at -80°C was calculated. Based on these, a difference between LRV of a virus titer in each specimen stored for 21 days at 25°C and LRV of a virus titer in each specimen stored for 21 days at -80°C was calculated for samples 1, 2, 3, 4 and 6. Table 4 shows the results. Further, Table 5 shows the results of calculating a difference between LRV of a virus titer in each specimen stored for 56 days at 4°C and LRV of a virus titer in each specimen stored for 56 days at -80°C for samples 1, 2, 3, 4 and 6.

### [Table 4]

**Table 4**

| | Isoelectric point of gelatin hydrolysate | Logarithmic reduction value (LRV) difference between LRV at 25°C and LRV at -80°C (3W) |
|---|---|---|
| Sample 1 | 4.7 | 0.7 |
| Sample 2 | 4.0 | 0.7 |
| Sample 3 | 4.3 | 1 |
| Sample 4 | 4.8 | 0.9 |
| Sample 6 | 8.0 | 4.8 |

### [Table 5]

**Table 5**

| | Isoelectric point of gelatin hydrolysate | Logarithmic reduction value (LRV) difference between LRV at 4°C and LRV at -80°C (8W) |
|---|---|---|
| Sample 1 | 4.7 | 0.3 |
| Sample 2 | 4.0 | 0.4 |
| Sample 3 | 4.3 | 0.5 |
| Sample 4 | 4.8 | 0.5 |
| Sample 6 | 8.0 | 1.2 |

From Tables 4 and 5 above, it can be evaluated that samples 1, 2, 3 and 4 enable a virus to be stably stored and transported at 4°C (refrigerated temperature) and 25°C (room temperature) because the LRV differences are not more than 1.0 log and not more than 1.5 log, respectively. In sample 6, the LRV difference exceeded 1.5 log at 25°C, and exceeded 1.0 log at 4°C.

### [Experiment 3: Study on suitable concentration of gelatin hydrolysate in virus stabilizer]

For samples 1, 7, 8, 9, 10, 11 and 12, TCID 50 of each of the specimens stored for 56 days at 4°C and for 21 days at 25°C was determined, and on the basis of the relevant value of TCID 50, LRV of a virus titer at 4°C and LRV of a virus titer at 25°C were calculated. Further, for samples 1, 7, 8, 9, 10, 11 and 12, TCID 50 of each of the specimens stored for 21 days and 56 days at -80°C was determined, and on the basis of the relevant value of TCID 50, LRV of a virus titer at -80°C was calculated. Based on these, a difference between LRV of a virus titer in each specimen stored for 21 days at 25°C and LRV of a virus titer in each specimen stored for 21 days at -80°C was calculated for samples 1, 7, 8, 9, 10, 11 and 12. Table 6 shows the results. Further, Table 7 shows the results of calculating a difference between LRV of a virus titer in each specimen stored for 56 days at 4°C and LRV of a virus titer in each specimen stored for 56 days at -80°C for samples 1, 7, 8, 9, 10, 11 and 12.

### [Table 6]

**Table 6**

| | Concentration of gelatin hydrolysate (mass%) | Logarithmic reduction value (LRV) difference between LRV at 25°C and LRV at -80°C (3W) |
|---|---|---|
| Sample 1 | 5 | 0.7 |
| Sample 7 | 0.1 | 1.8 |
| Sample 8 | 0.5 | 2 |
| Sample 9 | 1 | 1.0 |
| Sample 10 | 2.5 | 0.9 |
| Sample 11 | 10 | 0.9 |
| Sample 12 | 20 | 1.3 |

### [Table 7]

**Table 7**

| | Concentration of gelatin hydrolysate (mass%) | Logarithmic reduction value (LRV) difference between LRV at 4°C and LRV at -80°C (8W) |
|---|---|---|
| Sample 1 | 5 | 0.3 |
| Sample 7 | 0.1 | 1.4 |
| Sample 8 | 0.5 | 1.1 |
| Sample 9 | 1 | 0.7 |
| Sample 10 | 2.5 | 0.4 |
| Sample 11 | 10 | 0.3 |
| Sample 12 | 20 | 0.2 |

From Tables 6 and 7 above, it can be evaluated that samples 1, 9, 10, 11 and 12 enable a virus to be stably stored and transported at 4°C (refrigerated temperature) and 25°C (room temperature) because the LRV differences are not more than 1.0 log and not more than 1.5 log, respectively. In both samples 7 and 8, the LRV difference exceeded 1.5 log at 25°C, and exceeded 1.0 log at 4°C.

### [Experiment 4: Comparison with conventional virus stabilizer]

For samples 1 and 13, TCID 50 of each of the specimens stored for 56 days at 4°C and for 21 days at 25°C was determined, and on the basis of the relevant value of TCID 50, LRV of a virus titer at 4°C and LRV of a virus titer at 25°C were calculated. Further, for samples 1 and 13, TCID 50 of each of the specimens stored for 21 days and 56 days at -80°C was determined, and on the basis of the relevant value of TCID 50, LRV of a virus titer at -80°C was calculated. Based on these, a difference between LRV of a virus titer in each specimen stored for 21 days at 25°C and LRV of a virus titer in each specimen stored for 21 days at -80°C was calculated for samples 1 and 13. Table 8 shows the results. Further, Table 9 shows the results of calculating a difference between LRV of a virus titer in each specimen stored for 56 days at 4°C and LRV of a virus titer in each specimen stored for 56 days at -80°C for samples 1 and 13.

### [Table 8]

**Table 8**

| | Peptide component in virus stabilizer | Logarithmic reduction value (LRV) difference between LRV at 25°C and LRV at -80°C (3W) |
|---|---|---|
| Sample 1 | Gelatin hydrolysate | 0.7 |
| Sample 13 | Albumin | 2 |

### [Table 9]

**Table 9**

| | Peptide component in virus stabilizer | Logarithmic reduction value (LRV) difference between LRV at 4°C and LRV at -80°C (8W) |
|---|---|---|
| Sample 1 | Gelatin hydrolysate | 0.3 |
| Sample 13 | Albumin | 1.2 |

From Tables 8 and 9 above, it can be evaluated that sample 1 can suppress a decrease in virus titer at 4°C (refrigerated temperature) and 25°C (room temperature) more than sample 13.

### [Experiment 5: Study on effect of virus stabilizer for each type of virus]

For samples 1, 14, 15, 16 and 17, TCID 50 of each specimen stored for 21 days at 25°C was determined, and on the basis of the relevant value of TCID 50, LRV of a virus titer at 25°C was calculated. Further, for samples 1, 14, 15, 16 and 17, TCID 50 of each specimen stored for 21 days at -80°C was determined, and on the basis of the relevant value of TCID 50, LRV of a virus titer at -80°C was calculated. Based on these, a difference between LRV of a virus titer in each specimen stored for 21 days at 25°C and LRV of a virus titer in each specimen stored for 21 days at -80°C was calculated for samples 1, 14, 15, 16 and 17. For sample 17, FFU was determined instead of TCID. Table 10 shows the results.

Further, for samples 1, 14, 15 and 16, TCID 50 of each of the specimens stored for 21 days, 56 days and 210 days at 4°C was determined, and on the basis of the relevant value of TCID 50, LRV of a virus titer at 4°C was calculated. For samples 1, 14, 15 and 16, TCID 50 of each of the specimens stored for 21 days, 56 days and 210 days at -80°C was also determined, and on the basis of the relevant value of TCID 50, LRV of a virus titer at -80°C was calculated. Based on these, a difference between LRV of a virus titer in each of the specimens stored for 21 days, 56 days and 210 days at 4°C and LRV of a virus titer in each of the specimens stored for 21 days, 56 days and 210 days at -80°C was calculated for samples 1, 14, 15 and 16. Table 11 shows the results.

### [Table 10]

**Table 10**

| | Type of virus | Logarithmic reduction value (LRV) difference between LRV at 25°C and LRV at -80°C (3W) |
|---|---|---|
| Sample 1 | BHV-1 | 0.7 |
| Sample 14 | PI-3 | 0.9 |
| Sample 15 | Reo-3 | 0.5 |
| Sample 16 | Ad5 | 0.2 |
| Sample 17 | rNDV-GFP | 0.1 |

### [Table 11]

**Table 11**

| | Type of virus | Logarithmic reduction value (LRV) difference between LRV at 4°C and LRV at -80°C | | |
|---|---|---|---|---|
| | | 3W | 8W | 30W |
| Sample 1 | BHV-1 | 0.1 | 0.3 | 0.8 |
| Sample 14 | PI-3 | 0.2 | 0.6 | 0.9 |
| Sample 15 | Reo-3 | 0.3 | 0.2 | 0.2 |
| Sample 16 | Ad5 | 0.1 | 0.1 | 0.6 |

From Tables 10 and 11, it can be evaluated that samples 1, 14, 15, 16 and 17 enable a virus to be stably stored and transported at 4°C (refrigerated temperature) and 25°C (room temperature) because the LRV differences are not more than 1.0 log and not more than 1.5 log, respectively. Tables 10 and 11 reveal that sample 1 and samples 14 to 16 which had maintained activity for up to 3 weeks at 25°C was capable of maintaining activity for up to 30 weeks at 4°C, and therefore sample 17 may be likely to similarly maintain activity for up to 30 weeks at 4°C (refrigerated temperature).

Given the results from experiments 1 to 5 described above, it can be evaluated that samples 1 to 4, samples 9 to 12 and samples 14 to 17 of Examples enable a virus to be stably stored and transported in an environment at 4°C for 21 days.

### [Experiment 6: Effect by repetition of freezing and thawing of virus stabilizer]

Sample 1 was frozen at -80°C, and then thawed in iced water. This operation was repeated three times. Every time sample 1 is thawed (at the first, second and third rounds of the freezing and thawing), the virus titer of sample 1 was measured, and a logarithmic reduction value (LRV) for comparison with the virus titer before the freezing and thawing of sample 1 was calculated. Table 12 shows the results.

### [Table 12]

**Table 12**

| | Type of virus | Logarithmic reduction value (LRV) relative to that before freezing and thawing | | |
|---|---|---|---|---|
| | | First round of freezing and thawing | Second round of freezing and thawing | Third round of freezing and thawing |
| Sample 1 | BHV-1 | 0.1 | 0.2 | 0.1 |

From Table 12 above, it can be evaluated that sample 1 can maintain the virus titer even when subjected to freezing and thawing because the LRV was maintained near 0 in all of the first to third rounds of freezing and thawing.

While embodiments and Examples of the present invention have been described above, it has been forecast from the beginning that the embodiments and Examples described above are combined as appropriate.

The embodiments and Examples disclosed here should be construed to be illustrative and nonrestrictive in all respects. The scope of the present invention is given not by the description above but by claims, and intended to include all changes within meanings and spectra equivalent to claims.

## Claims

1. A virus stabilizer comprising a gelatin hydrolysate and an aqueous medium,
the virus stabilizer containing the gelatin hydrolysate at not less than 1 mass% and not more than 20 mass%,
the gelatin hydrolysate having a weight average molecular weight of not more than 10000,
the gelatin hydrolysate having an isoelectric point pH of not less than 4.0 and not more than 7.0.

2. The virus stabilizer according to claim 1, wherein the gelatin hydrolysate has a weight average molecular weight of not more than 6000.

3. The virus stabilizer according to claim 1 or 2, wherein the gelatin hydrolysate is a hydrolysate of alkali-treated gelatin.

4. The virus stabilizer according to any one of claims 1 to 3, wherein the virus stabilizer contains the gelatin hydrolysate at more than 2 mass% and not more than 10 mass%.

5. The virus stabilizer according to any one of claims 1 to 4, wherein the aqueous medium contains a salt having a buffering action.

6. The virus stabilizer according to any one of claims 1 to 5, wherein the aqueous medium contains at least one saccharide selected from the group consisting of sucrose, lactose, sorbitol, inositol, trehalose, mannitol, maltitol, xylitol, erythritol and glycerol.

7. The virus stabilizer according to any one of claims 1 to 6, wherein the aqueous medium contains at least one amino acid selected from the group consisting of methionine, arginine, tryptophane, glutamine and glutamic acid.

8. A gelatin hydrolysate for a virus stabilizer, having a weight average molecular weight of not more than 10000 and an isoelectric point pH of not less than 4.0 and not more than 7.0.

9. The gelatin hydrolysate for a virus stabilizer according to claim 8, wherein the gelatin hydrolysate for a virus stabilizer is liquid or powder.

10. A virus-containing composition, comprising the virus stabilizer according to any one of claims 1 to 7 and a virus.

11. The virus-containing composition according to claim 10, wherein the virus comprises at least one selected from the group consisting of a DNA virus having an envelope, a DNA virus having no envelope, an RNA virus having an envelope, and an RNA virus having no envelope.

12. The virus-containing composition according to claim 10 or 11, wherein the virus is a transgenic virus.

13. The virus-containing composition according to any one of claims 10 to 12, wherein in the virus-containing composition, a difference between the logarithmic reduction value of a virus titer in the case of storage at 25°C for 21 days and the logarithmic reduction value of a virus titer in the case of storage at -80°C for 21 days is not more than 1.5 log.

14. The virus-containing composition according to any one of claims 10 to 13, wherein in the virus-containing composition, a difference between the logarithmic reduction value of a virus titer in the case of storage at 4°C for 210 days and the logarithmic reduction value of a virus titer in the case of storage at -80°C for 210 days is not more than 1.0 log.

15. The virus-containing composition according to any one of claims 10 to 14, wherein in the virus-containing composition, the logarithmic reduction value when freezing and thawing is repeated three times is not more than 0.3 log.
